# EUROPEAN PATENT SPECIFICATION

(11) **EP 2 712 599 B1**
(45) Date of publication and mention of the grant of the patent: **25.05.2016**
(21) Application number: 13180566.5
(22) Date of filing: 15.08.2013
(51) Int. Cl.: A61H 9/00

(54) **Compression device pumping**
Druckvorrichtungspumpen
Pompage de dispositif de compression

(30) Priority: 28.09.2012 US 201213630829
(43) Date of publication of application: 02.04.2014
(73) Proprietor: Covidien LP, Mansfield, MA 02048 (US)
(72) Inventor: Deshpande, Manish, Canton, MA Massachusetts 02021 (US); Malhi, Arnaz, Watertown, MA Massachusetts 02472 (US); Hutton, Daniel, Brighton, MA Massachusetts 02135 (US)
(74) Representative: Gray, James

(56) References cited:
- WO-A1-2005/051283
- WO-A1-2007/085817
- US-A1- 2011 040 220
- US-A1- 2012 089 059

## Description

### TECHNICAL FIELD

The present disclosure generally relates to compression devices, and in particular to pumping fluid to and from compression devices.

### BACKGROUND

Intermittent pneumatic compression (IPC) devices are used to improve circulation and minimize the formation of thrombi in the limbs of patients by applying compression treatment to the limb through a series of compression cycles. A compression garment that can be worn on a limb of a patient includes one or more inflatable bladders positioned to apply compression to the limb when the garment is being worn and one or more bladders in the garment are inflated. Some compression devices include pumps that use solenoid valves to deliver pressurized fluid to the bladder in the garment. Diaphragm pumps require an electric motor and other associated mechanical mechanisms to convert rotational motion into reciprocating motion of a diaphragm. One reason these types of pumps are used for compression devices is that their relatively high flow rates (between about 5-8 Pa·m³/S (3-5 slpm) at 7 kPa (1 psi) of backpressure) are generally sufficient to meet the fluid flow demands of a conventional compression garment.

A single pump is most commonly mounted in a controller that is separate from the compression garment. The controller is typically mounted on a bed or other support next to the patient and tubing carries the compressed air from the controller to the garment. The tubing can be at a minimum a nuisance and may also lead to a loss of full function of the compression device if the tubing becomes kinked or is laid upon by the patient.

US2011/040220 discloses an apparatus and method for deep vein thrombosis phrophylaxis.

Another compression device is disclosed in US 2012/0089059.

### SUMMARY

In a first aspect, a compression device as claimed may generally comprise a compression garment positionable on the limb of the wearer and including an inflatable bladder for providing compression treatment to the limb. A pump assembly may be supported by the compression garment. The pump assembly may be in fluid communication with the bladder for pressurized fluid delivery. The pump assembly may comprises at least first and second pumps. Passaging may connect each of the first and second pumps for fluid communication with the inflatable bladder.

In said first aspect, the pumps may be plumbed to each other in at least one of a parallel configuration and a series configuration.

In said first aspect, a valve may be in fluid communication with each of the first and second pumps. The valve may be operable to selectively connect the first and second pumps in fluid communication with one another in parallel and to selectively connect the first and second pumps in fluid communication with one another in series.
In said first aspect, a controller may be supported by the compression garment, the controller controlling the valve.

In said first aspect, the controller may be configured to fluidly connect the first and second pumps in parallel when a pressure in the inflatable bladder is equal to or below a predetermined threshold and to fluidly connect the first and second pumps in series when the pressure in the inflatable bladder exceeds the predetermined threshold.
In said first aspect, the pump assembly may further comprise a third pump in fluid communication in parallel with the first pump. The valve may be adapted to selectively fluidly connect the first and third pumps in fluid communication in series with the second pump and to selectively connect the first and third pumps in fluid communication in parallel with the first pump.

In said first aspect, a controller may control the valve.

In said first aspect, the first and second pumps may each comprise a housing defining an inlet manifold and an outlet manifold. A nipple may project from one of the inlet and outlet manifolds. A first port may communicate with the inlet manifold and a second port may communicate with the outlet manifold. The nipple of the first pump may be adapted for selective sealing reception in the first port of the second pump and in the second port of the second pump.

In said first aspect, the first and second pumps may each be piezoelectric pumps.

In a second aspect, a method of delivering pressurized fluid to a compression garment may generally comprise operating at least two pumps of a pump assembly during a compression cycle in a first configuration for delivering pressurized fluid to a compression garment during the compression cycle for compressing a part of a wearer's body. During the compression cycle, the first arrangement may be changed so that the at least two pumps are arranged in a second arrangement, different from the first arrangement, for delivering pressurized fluid to the compression garment.

In said second aspect, in the first configuration, the at least two pumps may be arranged in one of series and parallel and, in the second configuration, the at least two pumps may be arranged in the other of series and parallel.

In said second aspect, operating the pumps in parallel when a pressure in the compression garment is equal to or below a predetermined threshold and operating the pumps in series when the pressure exceeds the predetermined threshold.

In said second aspect, the predetermined threshold may be about 8 kPa (60 mmHg).

In said second aspect, operating the at least two pumps in the first configuration may comprise moving a valve to one position and operating the at least two pumps in the second configuration may comprise moving the valve to another position different from said one position.

In said second aspect, in the first configuration, two pumps of the pump assembly may be arranged in parallel, and in the second configuration the two pumps may be placed in fluid communication with a third pump such that the two pumps in parallel are arranged in series with the third pump.

In said second aspect, operating the pumps in the first configuration when a pressure in the compression garment is below about 6.7 kPa (50 mmHg), and operating the pumps in the second configuration when the pressure in the inflation garment exceeds about 6.7 kPa (50 mmHg).

In a third aspect, a modular pump assembly for use in a compression device may generally comprise a first modular pump including a housing defining an inlet manifold and an outlet manifold. A pumping unit may be disposed for receiving fluid from the inlet manifold and exhausting fluid into the outlet manifold. A nipple may project from one of the inlet and outlet manifolds. A first port may communicate with the inlet manifold and a second port may communicate with the outlet manifold. A second modular pump may include a housing defining an inlet manifold and an outlet manifold. A pumping unit may be disposed for receiving fluid from the inlet manifold and exhausting fluid into the outlet manifold. A nipple may project from one of the inlet and outlet manifolds. A first port may communicate with the inlet manifold and a second port may communicate with the outlet manifold. The nipple of the first pump may be adapted for selective sealing reception in the first port of the second pump or in the second port of the second pump. The nipple of the second pump may be adapted for selective sealing reception in the first port of the first pump or in the second port of the first pump.

In said third aspect, the first pump may comprise a valve located in one of the first and second ports thereof and the second pump may comprise a valve located in one of the first and second ports of the second pump.

In said third aspect, the valve of the second pump may be disposed in the second port of the second pump. The nipple of the first pump may be configured to open the valve of the second pump upon insertion of the nipple of the first pump into the second port of the second pump, placing the outlet manifold of the first pump in fluid communication with the outlet manifold of the second pump.

Other objects and features will be apparent from the drawings and description and from the claims.

### BRIEF DESCRIPTION OF THE DRAWINGS

Fig. 1 is a block diagram of a compression device.
Fig. 2 is a schematic of a modular pump of the compression device of Fig. 1.
Fig. 3A is a schematic of a modular pump assembly including two modular pumps in series.
Fig. 3B is a schematic of a modular pump assembly including two modular pumps in parallel.
Fig. 4 is a schematic of an out-of-plane configuration of a modular pump.
Fig. 5 is a graph illustrating flow rate of various pump assemblies over a pressure range.
Figs. 6A-6E are schematics of different pump arrangements.
Fig. 7 is a graph illustrating flow rate of various pump assemblies over a pressure range.
Fig. 8A is a schematic of a two-pump assembly including a three way valve in communication with the pumps.
Fig. 8B is a schematic of a three-pump assembly including a three way valve in communication with the pumps.

Corresponding reference characters indicate corresponding parts throughout the drawings.

### DETAILED DESCRIPTION

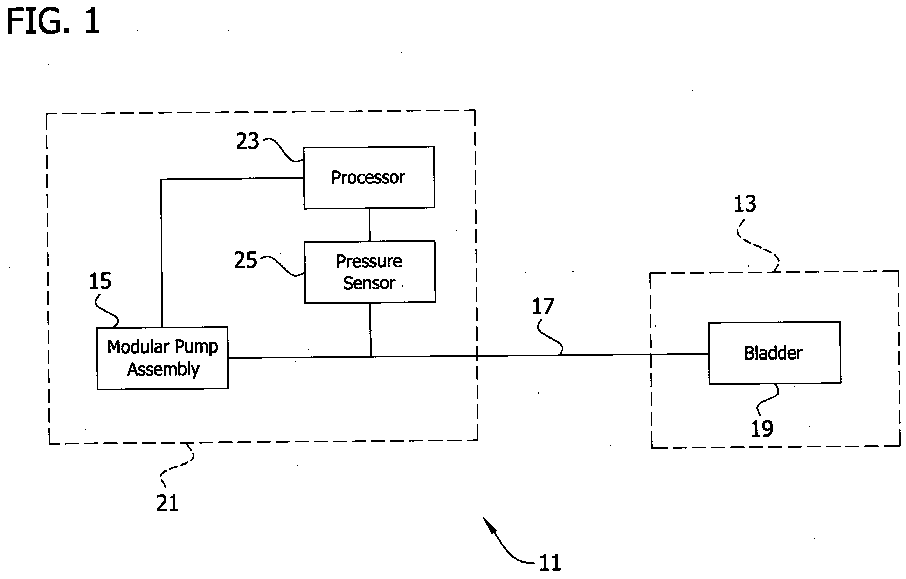

Referring to Figs. 1-2, a compression device 11 applies repeated, sequential compression therapy to a limb of a wearer. The compression device 11 includes a garment 13 sized and shaped to be wrapped around a leg or other limb of the wearer. A pump assembly 15 is fluidly connected to the garment 13 through conduit 17 for selectively pressurizing a bladder 19 of the garment by introducing gas (e.g., air) into the bladder. A controller 21 includes a processor 23 operatively connected to the pump assembly 15 for controlling the pressurization of the garment 13. A pressure sensor 25 is operatively connected to the processor 23 and coupled to the bladder 19 through the conduit 17 for measuring pressure in the bladder. Although a single bladder 19 is illustrated, the garment 13 can have two or more bladders. Moreover, while the conduit 17 and the controller 21 are shown as being incorporated into the garment 13, a controller and/or tubing may be separate from the garment and bladder.

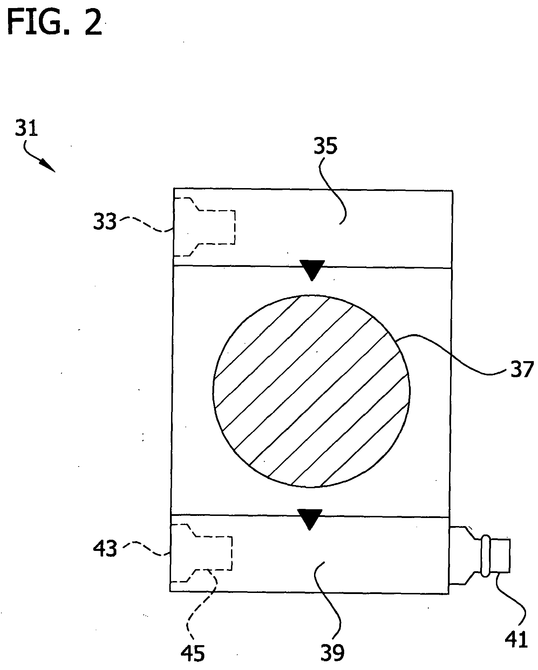

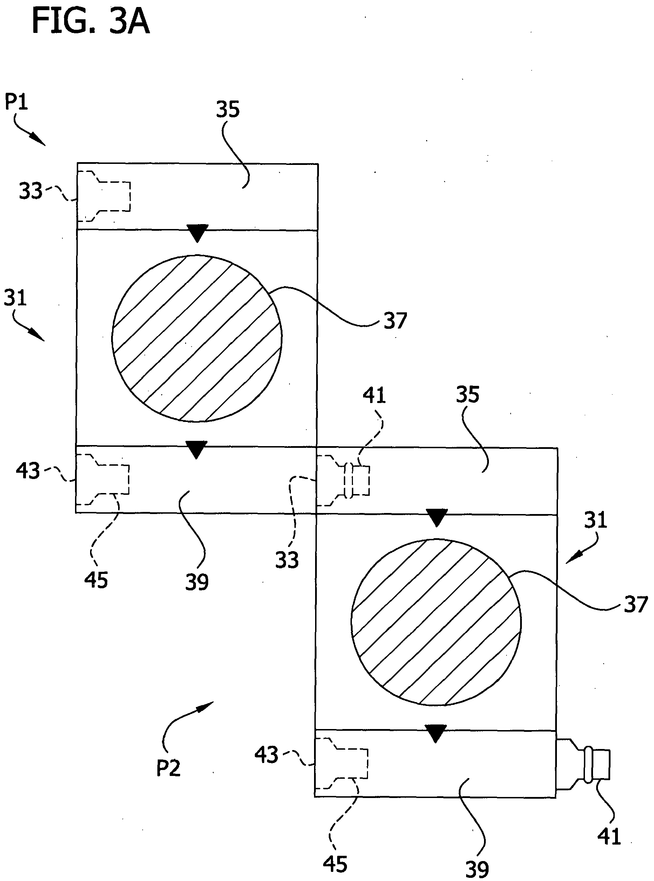

The modular pump assembly 15 may include two or more modular pumps 31, one of which is schematically illustrated in Fig. 2. The modular pump includes a first port 33 leading to an inlet manifold 35, a pumping unit 37 in fluid communication with the inlet manifold, an outlet manifold 39 in fluid communication with the pumping unit, and an outlet including a nipple 41. A second port 43 is located in the outlet manifold 39 on an end opposite the outlet nipple 41. A valve 45 can be disposed in the outlet manifold 39 to prevent fluid from escaping (or entering) the outlet manifold through the second port 43, as will be explained in greater detail below. The modular pump 31 can be micro pump, such as a piezoelectric pump, capable of about 1 slpm of flow under a backpressure of about 7 kPa (1 psi). Additionally or alternatively, the modular pump 31 can be another type of micropump (e.g., diaphragm, gear, piston, peristaltic, electroosmotic, electrohydrodynamic, magnetic, etc.). Moreover, it should be appreciated that that modular pump 31 can be a type of pump that is not a micropump. Still further, the pumps are shown as modular (e.g., Figs. 3A-4), non-modular pumps may be used such that the pumps may be plumbed together in a fixed arrangement.

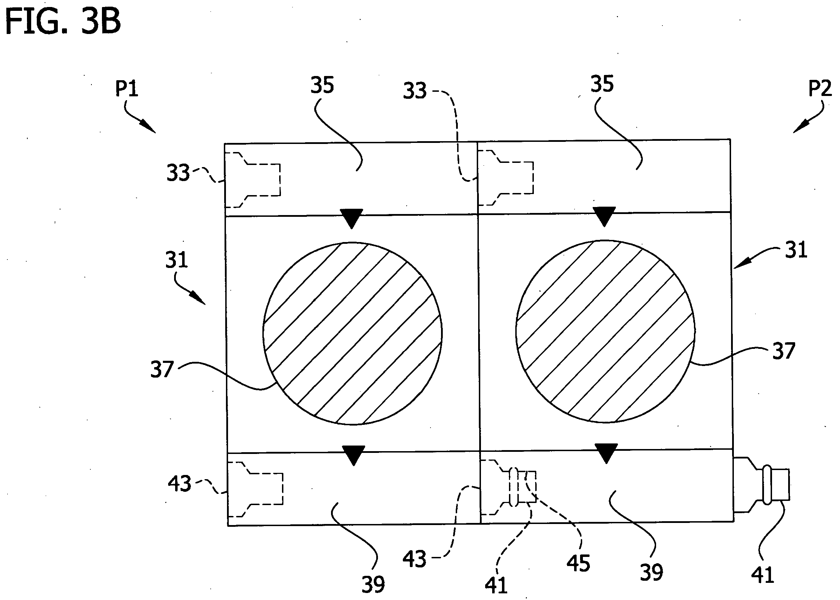

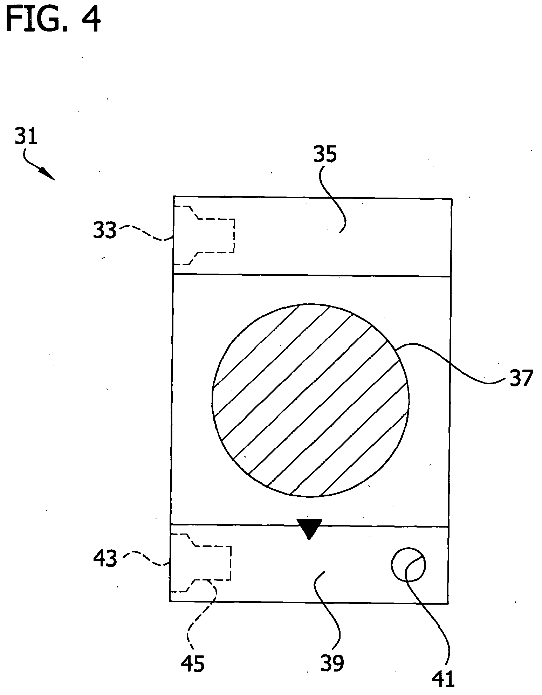

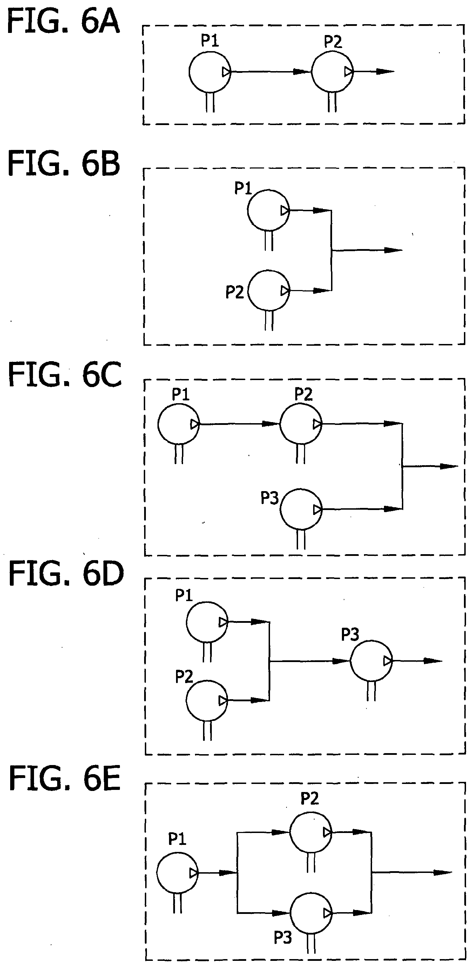

Conventional compression devices typically use diaphragm pumps capable of between about 5-8 Pa·m³/s (3-5 slpm) of flow at 7 kPa (1 psi) of backpressure. However, a single modular pump, which cannot operate in this range, may not be sufficient to meet the pressure requirements of a conventional compression device. To meet these pressure requirements, multiple modular pumps are combined to dynamically increase the overall flow rate of the pumps in a scalable and/or incremental manner. The modular pumps 31 can be combined in a variety of ways. For example, the pumps 31 can be combined in series such that an outlet nipple 41 of a first pump P1 is connected to the first port 33 of a second pump P2 (Figs. 3A and 6A). As another example, the pumps 31 can be combined in parallel such that the outlet nipple 41 of the first pump is connected to the second port 43 of the second pump (Figs. 3B and 6B). The valve 45 of the second pump P2. prevents fluid from escaping the second port 43 when the pumps are connected in series. The valve can be an elastomeric (e.g., silicone) membrane which has slits such that insertion of nipple 41 opens the valve for pneumatic communication. Any number of pumps can be combined in series and/or parallel subject to the structural and operational limits of the pump design. The pumps are shown such that manifolds are "in-plane" (i.e., inlet and outlet of the pump extend in the same direction). However, the pumps could be configured such that the manifolds of a given pump are out of plane. In the out of plane configuration, the outlet 41 of a pump 31 can be turned to be, for example, orthogonal to the inlet 33 (Fig. 4). In other examples, the outlet 41 can be disposed relative to the inlet 33 at an angle other than 90 degrees. This out of plane configuration can, for example, make combining the pumps easier and provide a more compact pump assembly.

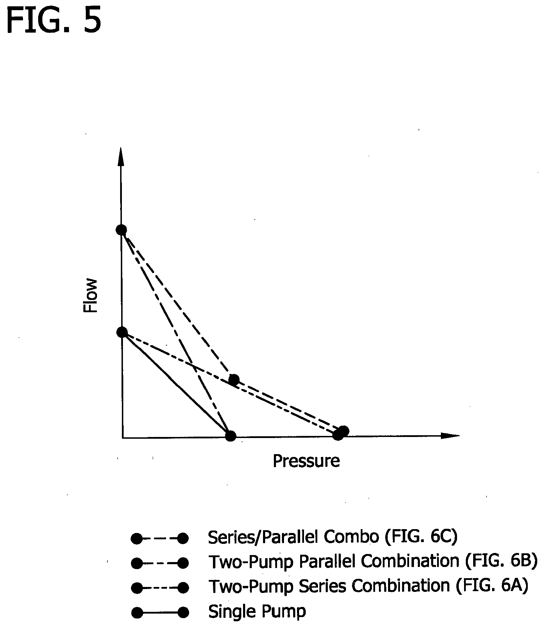

Referring now to Figure 5, an experimentally determined comparison of flow rate versus pressure for a single modular pump and various series and parallel pump combinations is shown. Generally, combining the pumps in series increases the operating range of the pumps (i.e., the pumps will operate at a higher backpressure than a single pump), but does not increase the maximum flow rate. However, the flow rate from the combined pumps in series does not diminish at increasing backpressure at the same rate as for a single pump so that higher flow rates may be attained through the range between the boundaries. Combining the pumps in parallel does not increase the overall pressure range of operation of the pump assembly as compared to a single pump, but increases the maximum flow output at lower backpressures. As shown in Fig. 5, the maximum output is nearly doubled for combined pumps in parallel as compared to a single pump, and remains higher at each pressure in the range until the maximum operating backpressure. Thus, in general, running the pump assembly in parallel increases the pneumatic output of the pump assembly at lower pressures, while at higher backpressures it is more beneficial to run the pump assembly in series.

As mentioned above, the pump assembly 15 can have configurations other than the two-pump series and parallel arrangements described above. For example, the pump assembly 15 may include three or more pumps arranged in various series and parallel configurations. Figure 6C shows a three-pump circuit including a first pump P1, a second pump P2, and a third pump P3. The first pump P1 is in series with pump P2. The series pumps P1, P2 are then together arranged in parallel with pump P3. Figure 5 shows that this configuration provides increased flow capacity in comparison to the two-pump configurations and the single pump over the entire working range of the pump assembly.

Figure 6D shows a three-pump circuit including two pumps P1, P2 in parallel with each other. An output manifold of the two pumps is in series with a third pump P3.

Figure 6E shows a three-pump circuit including a first pump P1 in series with an inlet manifold of second and third pumps P2, P3 which are in parallel with each other.

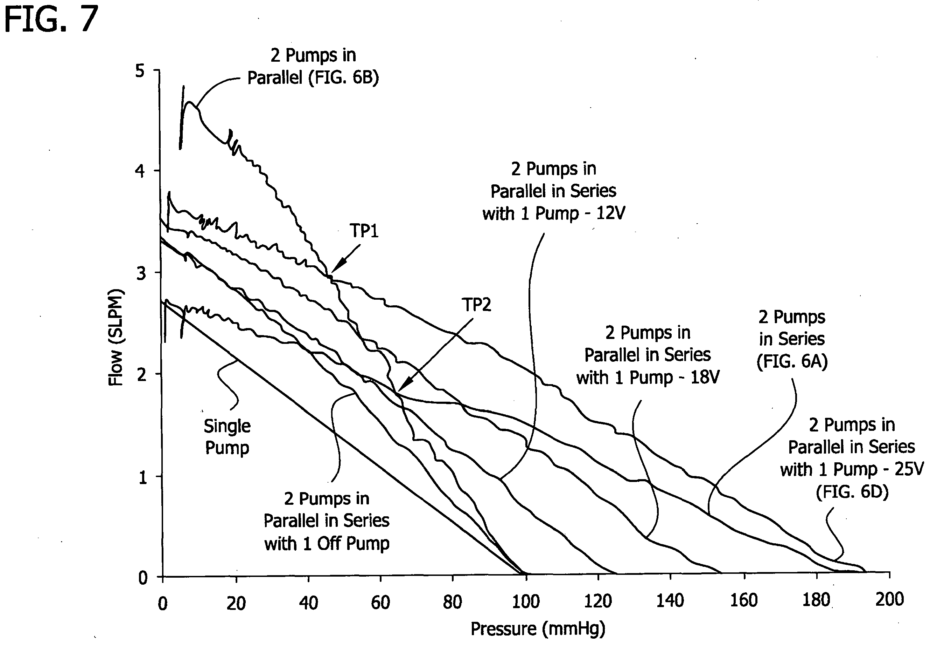

Fig. 7 shows the experimentally determined flow rates of the pump circuits shown in Figs. 6A, 6B, and 6D over a pressure range of 0-26.7 kPa (0-200 mmHg). Fig. 7 also shows flow profiles for the pump circuit shown in Fig. 6D with the third pump P3 in various operating configurations (off, 12V, 18V, 25V). The results shown in Fig. 7 indicate that, depending on the fluid pressure in the device, it may be desirable to use different pump arrangements to maximize flow output.

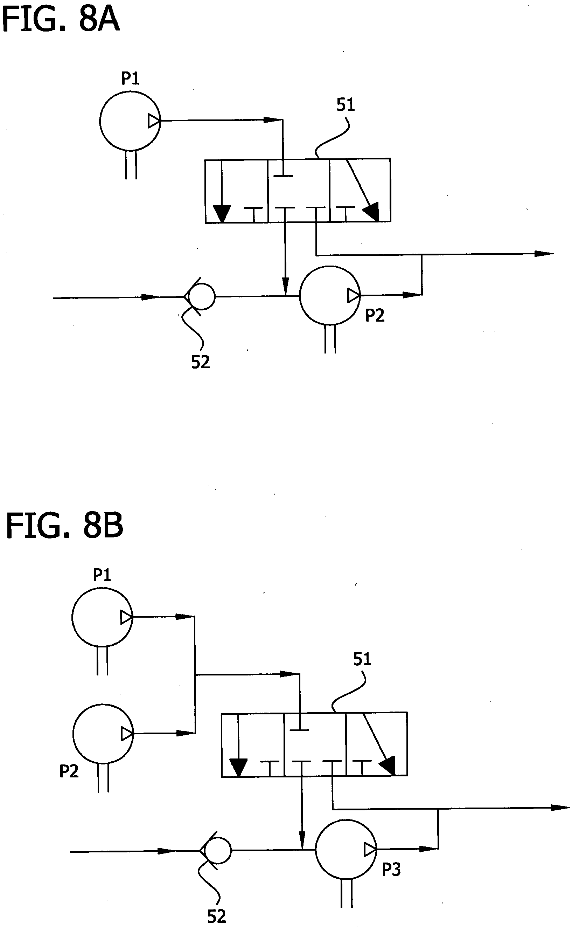

To take advantage of the varying fluid flow capabilities of the disclosed configurations, it is possible to construct a pump assembly that can switch between the disclosed configurations. For instance, a valve 51 (Fig. 8A) can be disposed in fluid communication between first and second pumps P1, P2 to selectively place pump P1 in series or in parallel with pump P2. The valve 51 can be switched to a first position where the outlet of pump P1 is fluidly connected to the inlet of pump P2 (series), or to a second position where the outlet of P1 is fluidly connected to an outlet of P2 via the second inlet (parallel). In the illustrated embodiment, the valve 51 is a 3-way/3-position piezo valve. A check valve 52 prevents the pneumatic output of P1 from being lost to the environment when pump P1 is in series with pump P2.

Referring to Fig. 7, it can be seen that a transition point TP2 indicates the pressure level where the performance of the two-pump parallel configuration falls below the two-pump series configuration.

The valve 51 can also be used to switch between the arrangements shown in Figs. 6B and 6D (see Fig. 8B). In the pump assembly configuration shown in Fig. 8B, first and second pumps P1, P2 are arranged in parallel and the valve 51 is disposed between the outlet of P1 and P2 and a third pump P3. The valve 51 can be switched to a first position where the outlet of P1 and P2 is fluidly connected to an outlet passage 53 bypassing P3 so that that the pump assembly is arranged in the two-pump parallel configuration shown in Fig. 6B. This configuration presupposes that P3 is turned to an off position. If P3 is turned on then the three pumps P1, P2, P3 will all be arranged in parallel. The valve 51 can also be switched to a second position where the outlet of P1 and P2 is fluidly connected to the inlet of P3, placing P3 in series with P1 and P2 and producing the pump assembly shown in Fig. 6D.

Referring to Fig. 7, it can be seen that a transition point TP1 indicates the pressure level where the performance of the two-pump parallel configuration in Fig. 6B falls below the configuration in Fig. 6D.

Therefore, during operation of the compression device 11, the processor 23 can operate the pump assembly 15 to switch between the various pump arrangements to optimize flow output over the entire pressure range based on feedback from the pressure transducer.

Referring again to the arrangement of Fig. 8B, as compression treatment is initiated (bladder pressure=0) and fluid is pumped into the bladder 19, the processor 23 can switch the valve 51 to the first position, placing pumps P1, P2 in parallel and bypassing pump P3 so that the device 11 is operating at an optimal flow capacity as pressure increases from 0 kPa (0 mmHg) (Fig. 7). Once the processor 23 determines that the pressure sensor 25 has measured a pressure in the bladder 19 exceeding a predetermined threshold (e.g., about 6.7 kPa (50 mmHg)), the processor 23 can switch the valve 51 to the second position, placing pumps P1 and P2 in series with pump P3 for superior performance in the higher pressure range. In summary, operation of the device 11 where the pressure in the bladder 19 is between about 0 and about 6.7 kPa (50 mmHg) (or initiation of a new cycle) causes the processor 23 to switch the pump assembly 15 to the two-pump parallel arrangement shown in Fig. 6B, and operation of the device where the pressure in the bladder exceeds about 6.7 kPa (50 mmHg) causes the processor to switch the pump assembly to the three-pump parallel/series configuration shown in Fig. 6D. It will be understood that this can be achieved by operating the valve 51 shown in Fig. 8B. As a result, flow output is optimized during the entire compression cycle for this pump assembly.

By way of another example, if the pump assembly has the configuration shown in Fig. 8A, where the valve 51 is between pumps P1 and P2, as compression treatment is initiated (pressure=0) and fluid is pumped into the bladder 19, the processor can switch the valve 51 to the second position placing the pumps P1, P2 in parallel so that the device 11 is operating at an optimal (high) flow capacity as pressure increases from 0 kPa (0 mmHg) (Fig. 7). The pressure sensor 25 monitors the pressure in the bladder 19 as compression treatment is continued. Once the processor 23 determines that the pressure sensor 25 has measured a pressure in the bladder 19 that exceeds a predetermined threshold (e.g., 8 kPa (60 mmHg)), the processor 23 can switch the valve 51 to the first position placing the pumps P1, P2 in series. The changeover occurs about at the point labeled TP2 in Fig. 7. Thus, operation of the device 11 where the pressure in the bladder 19 is between about 0 and about 8 kPa (60 mmHg) (or upon initiation of the new cycle) causes the processor 23 to switch the pump assembly 15 to the parallel arrangement (Fig. 6B), and operation of the device where the pressure in the bladder exceeds about 8 kPa (60 mmHg) signals to the processor to switch the pump assembly to the series arrangement (Fig. 6A). The changeover occurs approximately where transition point 2 (TP2) is identified on Fig. 7. It is to be understood that the change in configuration between Figs. 6B and 6A can be achieved by operation of the valve 51 shown in Fig. 8A. As a result, flow output is optimized during the entire compression cycle. Other possible ways of controlling or setting the configuration of the pumps may additionally or alternatively be used. In Fig. 7, four results for using two pumps

in parallel with each other in combination with a third pump in series are shown. The difference between these four results is the operating strength of the third pump (i.e., 0V, 12V, 18V or 25V).
Modifications and variations are possible without departing from the scope of this disclosure.

When introducing elements in the present disclosure, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects are achieved and other advantageous results attained.

As various changes could be made in the above constructions and methods without departing from the scope of the disclosure, it is intended that all matter contained in the above description and shown in the accompanying drawings shall be interpreted as illustrative and not in a limiting sense.

## Claims

1. A compression device (11) comprising:
a compression garment (13) positionable on the limb of the wearer, the garment comprising an inflatable bladder (19) for providing compression treatment to the limb;
a pump assembly (15) supported by the compression garment (13), the pump assembly (15) in fluid communication with the bladder (19) for pressurized fluid delivery; **characterized in that** the compression device (11) further comprises the pump assembly (15) comprising at least first and second pumps (31); and
passaging (17) connecting each of the first and second pumps for fluid communication with the inflatable bladder.

2. A compression device (11) as set forth in claim 1 wherein the pumps (31) are plumbed to each other in at least one of a parallel configuration and a series configuration.

3. A compression device (11) as set forth in claim 2 further comprising a valve (51) in fluid communication with each of the first and second pumps (31), the valve (51) operable to selectively connect the first and second pumps (31) in fluid communication with one another in parallel and to selectively connect the first and second pumps (31) in fluid communication with one another in series.

4. A compression device (11) as set forth in claim 3 further comprising a controller (21) supported by the compression garment (13), the controller (21) controlling the valve (51).

5. A compression device (11) as set forth in claim 4 wherein the controller (21) is configured to fluidly connect the first and second pumps (31) in parallel when a pressure in the inflatable bladder (19) is equal to or below a predetermined threshold and to fluidly connect the first and second pumps (31) in series when the pressure in the inflatable bladder (19) exceeds the predetermined threshold.

6. A compression device (11) as set forth in claim 3 wherein the pump assembly (15) further comprises a third pump in fluid communication in parallel with the first pump, the valve (51) being adapted to selectively fluidly connect the first and third pumps in fluid communication in series with the second pump and to selectively connect the first and third pumps in fluid communication in parallel with the first pump.

7. A compression device (11) as set forth in claim 6 further comprising a controller (21) for controlling the valve (51).

8. A compression device (11) as set forth in any preceding claim wherein the first and second pumps (31) each comprise a housing defining an inlet manifold (35) and an outlet manifold (39), a nipple (41) projecting from one of the inlet and outlet manifolds (35, 39), and a first port (33) communicating with the inlet manifold (35) and a second port (43) communicating with the outlet manifold (39), the nipple (41) of the first pump being adapted for selective sealing reception in the first port (33) of the second pump and in the second port (43) of the second pump.

9. A compression device (11) as set forth in any preceding claim wherein the first and second pumps (31) are each piezoelectric pumps.

## Patentansprüche

1. Kompressionsvorrichtung (11), umfassend:
ein Kompressionskleidungsstück (13), das an der Gliedmaße des Trägers positionierbar ist, wobei das Kleidungsstück einen aufblasbaren Balg (19) zum Bereitstellen von Kompressionsbehandlung für die Gliedmaße umfasst;
eine Pumpenbaugruppe (15), die von dem Kompressionskleidungsstück (13) getragen wird, wobei die Pumpenbaugruppe (15) zur Abgabe eines Druckfluids in Fluidverbindung mit dem Balg (19) steht; **dadurch gekennzeichnet** die Kompressionsvorrichtung (11) ferner die Pumpenbaugruppe (15), die wenigstens eine erste und zweite Pumpe (31) umfasst; und
Durchlässe (17) umfasst, die jeweils die erste und die zweite Pumpe zur Fluidverbindung mit dem aufblasbaren Balg verbinden.

2. Kompressionsvorrichtung (11) nach Anspruch 1, wobei die Pumpen (31) in wenigstens einer von einer parallelen Konfiguration und einer Reihenkonfiguration fest aneinander angeschlossen sind.

3. Kompressionsvorrichtung (11) nach Anspruch 2, ferner umfassend ein Ventil (51) in Fluidverbindung mit jeder der ersten und zweiten Pumpe (31), wobei das Ventil (51) betriebsfähig ist, um selektiv die erste und zweite Pumpe (31) in Fluidverbindung parallel miteinander zu verbinden und selektiv die erste und zweite Pumpe (31) in Fluidverbindung in Reihe miteinander zu verbinden.

4. Kompressionsvorrichtung (11) nach Anspruch 3, ferner umfassend eine Steuereinrichtung (21), die von dem Kompressionskleidungsstück (13) getragen wird, wobei die Steuereinrichtung (21) das Ventil (51) steuert.

5. Kompressionsvorrichtung (11) nach Anspruch 4, wobei die Steuereinrichtung (21) dazu konfiguriert ist, die erste und zweite Pumpe (31) parallel in Fluidverbindung miteinander zu setzen, wenn ein Druck in dem aufblasbaren Balg (19) gleich oder unter einem vorgegebenen Schwellenwert ist, und die erste und zweite Pumpe (31) in Reihe in Fluidverbindung miteinander zu setzen, wenn der Druck in dem aufblasbaren Balg (19) den vorgegebenen Schwellenwert übersteigt.

6. Kompressionsvorrichtung (11) nach Anspruch 3, wobei die Pumpenbaugruppe (15) ferner eine dritte Pumpe umfasst, die parallel in Fluidverbindung mit der ersten Pumpe steht, wobei das Ventil (51) dazu angepasst ist, selektiv die erste und dritte Pumpe in Reihe mit der zweiten Pumpe fluidisch in Fluidverbindung zu setzen und selektiv die erste und dritte Pumpe parallel mit der ersten Pumpe in Fluidverbindung zu setzen.

7. Kompressionsvorrichtung (11) nach Anspruch 6, ferner umfassend eine Steuereinrichtung (21) zum Steuern des Ventils (51).

8. Kompressionsvorrichtung (11) nach einem der vorangehenden Ansprüche, wobei die erste und zweite Pumpe (31) jeweils ein Gehäuse, das einen Einlassverteiler (35) und einen Auslassverteiler (39), definiert, einen Nippel (41), der von einem von dem Einlass- und Auslassverteiler (35, 39) vorspringt, und einen ersten Anschluss (33), der mit dem Einlassverteiler (35) in Verbindung steht, und einen zweiten Anschluss (43) umfassen, der mit dem Auslassverteiler (39) in Verbindung steht, wobei der Nippel (41) der ersten Pumpe dazu angepasst ist, selektiv die Aufnahme im ersten Anschluss (33) der zweiten Pumpe und in dem zweiten Anschluss (43) der zweiten Pumpe zu verschließen.

9. Kompressionsvorrichtung (11) nach einem der vorangehenden Ansprüche, wobei die erste und zweite Pumpe (31) jeweils piezoelektrische Pumpen sind.

## Revendications

1. Dispositif de compression (11) comprenant :
un vêtement de compression (13) positionnable sur le membre du porteur, le vêtement comprenant une poche gonflable (19) pour fournir un traitement de compression au membre ;
un ensemble de pompage (15) supporté par le vêtement de compression (13), l'ensemble de pompage (15) en communication fluidique avec la poche (19) pour la fourniture de fluide sous pression ; **caractérisé en ce que** le dispositif de compression (11) comprend en outre l'ensemble de pompage (15) comprenant au moins des première et deuxième pompes (31) ; et le passage (17) reliant chacune des première et deuxième pompes pour la communication fluidique avec la poche gonflable.

2. Dispositif de compression (11) selon la revendication 1, dans lequel les pompes (31) sont plombées l'une l'autre dans au moins une d'une configuration parallèle et une configuration en série.

3. Dispositif de compression (11) selon la revendication 2, comprenant en outre une valve (51) en communication fluidique avec chacune des première et deuxième pompes (31), la valve (51) servant à relier sélectivement les première et deuxième pompes (31) en communication fluidique l'une avec l'autre parallèlement et à relier sélectivement les première et deuxième pompes (31) en communication fluidique l'une avec l'autre en série.

4. Dispositif de compression (11) selon la revendication 3, comprenant en outre un contrôleur (21) supporté par le vêtement de compression (13), le contrôleur (21) contrôlant la valve (51).

5. Dispositif de compression (11) selon la revendication 4, dans lequel le contrôleur (21) est configuré pour relier fluidiquement les première et deuxième pompes (31) parallèlement lorsqu'une pression dans la poche gonflable (19) est égale ou inférieure à un seuil prédéterminé et pour relier fluidiquement les première et deuxième pompes (31) en série lorsque la pression dans la poche gonflable (19) excède le seuil prédéterminé.

6. Dispositif de compression (11) selon la revendication 3, dans lequel l'ensemble de pompage (15) comprend en outre une troisième pompe en communication fluidique parallèlement à la première pompe, la valve (51) étant adaptée pour relier fluidiquement sélectivement les première et troisième pompes en communication fluidique en série avec la deuxième pompe et pour relier sélectivement les première et troisième pompes en communication fluidique en parallèle avec la première pompe.

7. Dispositif de compression (11) selon la revendication 6, comprenant en outre un contrôleur (21) pour contrôler la valve (51).

8. Dispositif de compression (11) selon une quelconque revendication précédente, dans lequel les première et deuxième pompes (31) comprennent chacune un boîtier définissant un collecteur d'entrée (35) et un collecteur de sortie (39), un nipple (41) faisant saillie d'un des collecteurs d'entrée et de sortie (35, 39) et un premier orifice (33) communiquant avec le collecteur d'entrée (35) et un second orifice (43) communiquant avec le collecteur de sortie (39), le nipple (41) de la première pompe étant adapté pour la réception étanche sélective dans le premier orifice (33) de la deuxième pompe et dans le second orifice (43) de la deuxième pompe.

9. Dispositif de compression (11) selon une quelconque revendication précédente, dans lequel les première et deuxième pompes (31) sont chacune des pompes piézoélectriques.
